# EUROPEAN PATENT APPLICATION

(11) **EP 1 815 869 A1**
(43) Date of publication of application: **08.08.2007**
(21) Application number: 05811357.2
(22) Date of filing: 24.11.2005
(51) Int. Cl.: A61K 47/34, A61K 9/107, A61K 31/7048

(54) **METHOD OF CHANGING MORPHOLOGY OF BLOCK COPOLYMER**

(30) Priority: 24.11.2004 JP 2004339341
(71) Applicant: NanoCarrier Co., Ltd., Kashiwa-shi, Chiba 277-0882 (JP)
(72) Inventor: YAMAMOTO, Yasuo c/o NanoCarrier Co., Ltd.,, Kashiwa-shi, Chiba 2770882 (JP); KOKUBO, Miho c/o NanoCarrier Co., Ltd.,, Kashiwa-shi, Chiba 2770882 (JP)
(74) Representative: Cohausz & Florack
(86) International application number: PCT/JP2005/022035
(87) International publication number: WO 2006/057429

(57) **Abstract**

The invention provides a pharmaceutical formulation comprising drug-encapsulated polymer micelles formed from a block copolymer having a hydrophilic segment and hydrophobic segment, and has been subjected to high-pressure treatment. The invention also provides a treatment method for a block copolymer having a hydrophilic segment and a hydrophobic segment for formation of drug-encapsulated polymer micelles, the treatment method comprises a step of subjecting the block copolymer to high-pressure treatment.

## Description

### Technical Field

The present invention relates to a pharmaceutical formulation comprising drug-encapsulated polymer micelles formed of a block copolymer having a hydrophilic segment and a hydrophobic segment and is characterized by being subjected to high-pressure treatment, and to a high-pressure treatment method for the block copolymer for production of such a pharmaceutical formulation.

### Background Art

The use of block copolymers, comprising both a hydrophilic segment and a hydrophobic segment, as drug carriers and methods of encapsulating certain drugs in polymer micelles formed by the copolymers are known (Japanese Unexamined Patent Publication (Kokai) No. 6-107565, USP 5,449,513). Also known are a composition comprising homogeneous polymer micelles encapsulating a water-insoluble drug, and a method for its preparation (Japanese Unexamined Patent Publication (Kokai) No. 11-335267). In particular, block copolymers composed of a polyethylene glycol hydrophilic segment and a polyamino acid hydrophobic segment have the ability to automatically form micelles in water, and therefore are suitable for drug delivery.

Block copolymers used for micelle formation assemble in water and form spherical shaped micelles with the ends of the hydrophobic segments pointing inward. The sizes of the micelles range from several nanometers to several hundred nanometers. Block copolymers with a molecularly uniform three-dimensional structure are advantageous because they can form homogeneous monodispersed micelles of uniform particle size.

However, products of synthesis and purification of ordinary block copolymers are purposely formed with various three-dimensional structures arising from polyamino acid segments, and they therefore include nonhomogeneous structures.

Consequently, when such block copolymers are used to form micelles, they are disadvantageous in that the resulting micelles have wide dispersibility or are separated into two or more peaks based on size, while their filterability is also significantly inferior. Such micelles are not suitable for drug delivery.

### DISCLOSURE OF THE INVENTION

The present invention provides a block copolymer having a morphology allowing formation of micelles suitable for drug delivery.

The present inventors have discovered that by subjecting a block copolymer having a hydrophilic segment and a hydrophobic segment to high-pressure treatment, the micelles formed are homogeneous and monodispersed with a uniform particle size, while their filterability is also highly satisfactory, and the invention has been completed upon this discovery.

According to the invention, polymer nano-micelles suitable for a drug delivery system can be provided and produced in mass.

The present invention encompasses the following aspects.
(1) A pharmaceutical formulation comprising drug-encapsulated polymer micelles formed from a block copolymer having a hydrophilic segment and hydrophobic segment, and has been subjected to high-pressure treatment.
(2) A pharmaceutical formulation according to (1), wherein the high-pressure treatment is carried out by a process including a step in which a solution containing the block copolymer is subjected to ultrahigh-pressure dispersion/emulsification.
(3) A pharmaceutical formulation according to (2), wherein the ultrahigh-pressure dispersion/emulsification is carried out with an ultrahigh-pressure homogenizer.
(4) A pharmaceutical formulation according to any one of (1)-(3), wherein the pressure for the high-pressure treatment is 3,000 MPa or higher.
(5) A pharmaceutical formulation according to (4), wherein the pressure for the high-pressure treatment is 20,000 MPa or higher.
(6) A pharmaceutical formulation according to (1)-(5), wherein the block copolymer which has a higher-order structure is subjected to high-pressure treatment to yield a polymer with the higher-order structure of the polymer destroyed.
(7) A pharmaceutical formulation according to (1)-(5), wherein the block copolymer which has a higher-order structure and is biocompatible is subjected to high-pressure treatment to yield a polymer with the higher-order structure of the polymer destroyed.
(8) A pharmaceutical formulation according to any one of (1)-(7), wherein the hydrophobic segment is a polyamino acid derivative.
(9) A pharmaceutical formulation according to (8), wherein the polyamino acid derivative is a poly(aspartic acid) derivative.
(10) A pharmaceutical formulation according to (9), wherein the polyamino acid derivative is a poly(glutamic acid) derivative.
(11) A pharmaceutical formulation according to any one of (1)-(7), wherein the hydrophobic segment is a biocompatible polymer or a biodegradable polymer.
(12) A pharmaceutical formulation according to any one of (1)-(11), wherein the hydrophilic segment is polyethylene glycol or a derivative thereof.
(13) A pharmaceutical formulation according to any one of (1)-(12), wherein the hydrophilic segment has 30-1000 repeating units and the hydrophobic segment has 10-100 repeating units.
(14) A pharmaceutical formulation according to any one of (1)-(13), which contains a drug selected from the group of drugs consisting of anticancer agents, immunosuppressive agents, antibiotics, antirheumatic drugs, antimicrobial agents, antihypertensive drugs, central nervous system acting drugs, hormone agents, diabetes drugs, anti-inflammatory drugs, analgesics, antiviral agents, antimalarial drugs, biopreparations, gene therapy agents such as DNA or RNA, antibody agents, proteins and peptides.
(15) A pharmaceutical formulation according to any one of (1)-(13), which comprises a drug selected from the group consisting of roxithromycin, paclitaxel, topotecan, camptothecin, cisplatin, daunorubicin hydrochloride, methotrexate, mitomycin C, docetaxel, vincristine sulfate, polyene antibiotics, amphotericin B, nystatin and prostaglandins.
(16) A treatment method for a block copolymer having a hydrophilic segment and a hydrophobic segment for formation of drug-encapsulated polymer micelles, the treatment method comprises a step of subjecting the block copolymer to high-pressure treatment.
(17) A method according to (16), wherein the high-pressure treatment is carried out by a process including a step in which a solution containing the block copolymer is subjected to ultrahigh-pressure dispersion/emulsification.
(18) A method according to (17), wherein the ultrahigh-pressure dispersion/emulsification is carried out with an ultrahigh-pressure homogenizer.
(19) A method according to any one of (16)-(18), wherein the pressure for the high-pressure treatment is 3,000 MPa or higher.
(20) A method according to (19), wherein the pressure for the high-pressure treatment is 20,000 MPa or higher.
(21) A method according to (16)-(20), wherein the block copolymer which has a higher-order structure is subjected to high-pressure treatment to yield a polymer with the higher-order structure of the polymer destroyed.
(22) A method according to (16)-(20), wherein the block copolymer which has a higher-order structure and is biocompatible is subjected to high-pressure treatment to yield a polymer with the higher-order structure of the polymer destroyed.
(23) A method according to any one of (16)-(22), wherein the hydrophobic segment is a polyamino acid derivative.
(24) A method according to (23), wherein the polyamino acid derivative is a poly(aspartic acid) derivative.
(25) A method according to (24), wherein the polyamino acid derivative is a poly(glutamic acid) derivative.
(26) A method according to any one of (16)-(22), wherein the hydrophobic segment is a biocompatible polymer or a biodegradable polymer.
(27) A method according to (16)-(26), wherein the hydrophilic segment is polyethylene glycol or a derivative thereof.
(28) A method according to any one of (16)-(27), wherein the hydrophilic segment has 30-1000 repeating units and the hydrophobic segment has 10-100 repeating units.
(29) A method according to any one of (16)-(28), which includes the use of a drug selected from the group of drugs consisting of anticancer agents, immunosuppressive agents, antibiotics, antirheumatic drugs, antimicrobial agents, antihypertensive drugs, central nervous system acting drugs, hormone agents, diabetes drugs, anti-inflammatory drugs, analgesics, antiviral agents, antimalarial drugs, biopreparations, gene therapy agents such as DNA or RNA, antibody agents, proteins and peptides.
(30) A method according to (16)-(28), which includes the use of a drug selected from the group consisting of roxithromycin, paclitaxel, topotecan, camptothecin, cisplatin, daunorubicin hydrochloride, methotrexate, mitomycin C, docetaxel, vincristine sulfate, polyene antibiotics, amphotericin B, nystatin and prostaglandins.

### Brief Description of the Drawings

Fig. 1 is a pair of Z-potential measurement graphs of (a) drug-encapsulated pressure-treated micelles and (b) drug-encapsulated untreated micelles (POWER SPECTRUM).
Fig. 2 is a photograph showing the conditions of solutions of a pressure-treated block copolymer and an untreated block copolymer.

### Best Mode for Carrying Out the Invention

The block copolymer used for the invention having a hydrophilic segment (hereinafter referred to as segment A) and a hydrophobic segment (hereinafter referred to as segment B) may be an "AB-type" or "ABA-type" block copolymer. The polymer composed of the hydrophilic segment is not limited, and there may be mentioned segments of polyethylene glycol, polyphosphoric acid, polyoxyethylene, polysaccharides, polyacrylamide, polyacrylic acid, polymethacrylamide, polymethacrylic acid, polyvinylpyrrolidone, polyvinyl alcohol, polymethacrylic acid ester, polyacrylic acid ester, polyamino acid, and derivatives thereof. Preferred among these are segments composed of polyethylene glycol. The hydrophilic segment may have a low molecular functional group on the opposite side of the end bonding with the hydrophobic segment, so long as it does not adversely affect formation of the polymer micelles.

The hydrophobic segment is also not limited, and there may be mentioned polypeptides, particularly polypeptides of polyhomoamino acids, and for example, L-or D-amino acids or their racemic mixtures, and especially L-amino acids such as poly(aspartic acid), poly(glutamic acid), polyaspartic acid esters, polyglutamic acid esters or their partial hydrolysates, polylysine, polyacrylic acid, polymethacrylic acid, polymalic acid, polylactic acid, polyalkylene oxides, long-chain alcohols, and other known biocompatible polymers, biodegradable polymers and the like. The hydrophobic segment may have a low molecular functional group on the opposite side of the end bonding with the hydrophilic segment, similar to that explained for the hydrophilic segment, so long as it does not adversely affect interaction between the drug and the hydrophobic segment during formation of the polymer micelles.

The hydrophilic segment and hydrophobic segment are not restricted in size so long as they can form polymer micelles in an aqueous solution (or aqueous medium) in the presence of a water-insoluble drug, but generally the hydrophilic segment has preferably 30-1000 and more preferably 50-600 repeating units, while the hydrophobic segment preferably has 10-100 and more preferably 15-80 repeating units.

The present invention exhibits an effect of increasing the dispersibility and filterability of micelles by performing high-pressure treatment of a micelle-forming block copolymer having a hydrophilic segment and a hydrophobic segment. Without being bound to any particular theory, it is conjectured that the high-pressure treatment improves the dispersibility and filterability of the micelles by altering, for example, destroying, the higher-order structure of the block copolymer.

The high-pressure treatment is preferably carried out by ultrahigh-pressure dispersion/emulsification of the block copolymer. The ultrahigh-pressure dispersion/emulsification treatment may be conducted using, for example, an ultrahigh-pressure homogenizer. A preferred example of an ultrahigh-pressure homogenizer is a microfluidizer (Microfluidics^{™} M-110-EH). The pressure for the ultrahigh-pressure dispersion/emulsification treatment is, for example, a pressure of 3,000 MPa or higher or 5,000 MPa or higher, preferably 10,000 MPa or higher and more preferably 20,000 MPa or higher. There is no particular upper limit on the pressure, but it is preferably not higher than 100,000 MPa in consideration of the load on the apparatus. The temperature for carrying out the ultrahigh-pressure dispersion/emulsification treatment is not particularly restricted, but it is preferably 0°C or higher, more preferably 4°C or higher, even more preferably 10°C or higher, especially 20°C or higher, especially preferably 25°C or higher and most preferably 30°C or higher, and 120°C or lower, more preferably 100°C or lower, even more preferably 80°C or lower, especially 70°C or lower, especially preferably 60°C or lower and most preferably 50°C or lower.

The pressurizing treatment time is not particularly restricted, but a time of about 1-30 minutes, for example, is suitable.

An aqueous dispersion is prepared containing the block copolymer treated in this manner and one or more optional auxiliary agents selected from the group consisting of saccharides, inorganic salts and polyethylene glycols. Saccharides used as such auxiliary agents are not particularly restricted, but there may be mentioned as preferable monosaccharides or oligosaccharides such as maltose, trehalose, xylitol, glucose, sucrose, fructose, lactose, mannitol and dextrin, or sugar alcohols. Any inorganic salts that are pharmaceutically acceptable may also be used, and preferred ones that may be mentioned include chlorides such as sodium chloride, potassium chloride, magnesium chloride and calcium chloride. As polyethylene glycols there may be mentioned polyethylene glycols with 20-800 and preferably 20-200 oxyethylene (-(OCH₂CH₂)-) units. Examples of such polyethylene glycols suitable for use include macrogols 1000, 1540, 4000, 6000, 20,000 and 35,000 which are listed in Iyakuhin Tenkabutsu Jiten [Dictionary of Drug Additives].

The dispersion may be prepared by simultaneously adding the block copolymer and auxiliary agents to water and stirring, or by first preparing an aqueous solution of the auxiliary agents and adding the block copolymer thereto, or by preparing a mixture in the reverse order and stirring. A conventional stirrer or ultrasonic waves may be used for the stirring. The dispersion is not particularly restricted, but usually it may contain the block copolymer at a concentration of 0.1-40% by mass, and optionally saccharides at a concentration of 0.5-80% by mass, polyethylene glycols at a concentration of 0.5-40% by mass and inorganic salts at 0.5-10% by mass.

The drug to be used for the invention may be any drug that can be encapsulated by the polymer micelles formed by the block copolymer, and it may be selected from among anticancer agents, immunosuppressive agents, antibiotics, antirheumatic drugs, antimicrobial agents, antihypertensive drugs, central nervous system acting drugs, hormone agents, diabetes drugs, anti-inflammatory drugs, analgesics, antiviral agents, antimalarial drugs, biopreparations, gene therapy agents such as DNA or RNA, antibody agents, proteins and peptides. Such drugs include, but are not limited to, cancer drugs comprising roxithromycin, paclitaxel, topotecan, camptothecin, cisplatin, daunorubicin hydrochloride, methotrexate, mitomycin C, docetaxel, vincristine sulfate and the like and their derivatives, polyene antibiotics, and fat-soluble drugs such as amphotericin B, nystatin and the like, or prostaglandins and their derivatives.

Particularly convenient for use in the method of the invention are roxithromycin, paclitaxel, topotecan and docetaxel.

According to the invention, such drugs may be prepared as organic solutions by being dissolved with non-water-miscible organic solvents. Such solvents are not particularly restricted, and there may be mentioned dichloromethane, chloroform, diethyl ether, dibutyl ether, ethyl acetate, butyl acetate and their mixtures.

The optimum drug concentration in the solution will differ depending on the solvent used and the combination of drugs, but generally it may be a concentration of 0.1-50% by mass. The mixing procedure may be carried out at room temperature or at a low temperature.

The aqueous dispersion and organic solution prepared in the manner described above are combined to prepare a mixture, which is then subjected to stirring treatment (which may include sonication) for a sufficient time to cause the drug to be encapsulated in the polymer micelles. The treatment may be carried out at room temperature or at a lower temperature (up to 5°C). The organic solvent may also be evaporated off during the stirring. The procedure described above yields a drug-containing polymer micelle dispersion, and if necessary the saccharides and polyethylene glycols mentioned above may be added to the dispersion, for example, to stabilize the drug-containing polymer micelles in the subsequent lyophilization treatment or to inhibit aggregation of the micelle particles. The saccharides and/or polyethylene glycols are preferably added to final concentrations of 0.1-80% by mass for saccharides and 0.5-40% by mass for polyethylene glycols, in cases where they are added during preparation of the polymer micelle dispersion, but they may be added in excess of these ranges so long as there is no adverse effect on preparation of the lyophilized drug-containing polymer micelles or reconstitution of the obtained lyophilized product with the aqueous medium. The aqueous medium referred to here may be purified water, deionized water, buffered water, isotonized water or the like. The pH during preparation of the formulation of the invention is preferably 4.0-7.5, and pH regulators, antioxidants (ascorbic acid, sodium ascorbate, sodium thiosulfate) and the like may be added as necessary.

The composition comprising the drug-containing polymer micelles provided in this manner contains monodispersed micelles with almost uniform particle sizes. The particle sizes of the formed micelles will depend on the size of the block copolymer, but may be in the range of preferably 10-200 nm and more preferably 20-180 nm. The composition comprising the drug-containing micelles may be prepared as a formulation for oral administration or a formulation for parenteral administration depending on the drug, but it is particularly advantageous as a formulation for parenteral administration. In the case of a formulation for parenteral administration, such as an injection, salts, saccharides or other compounds may be added as necessary for isotonicity in blood, the drug-containing polymer micelles may be mixed in a liquid diluent commonly used in pharmaceutical formulations such as water, ethyl alcohol or propylene glycol, or if necessary an antisettling agent such as ethoxylated isostearyl alcohol or polyoxyethylene sorbitol may be added.

The drug-containing polymer micelle dispersion may be optionally subjected to sonication, as well as filtration treatment if necessary. The sonication is not particularly restricted, and for example, it may be carried out for between 1 second and 2 hours and preferably between 1 minute to 1 hour, at 1-200 W and preferably 10-50 W. The filtration treatment is also not particularly restricted, and for example, a 0.45 µm and preferably 0.22 µm hydrophilic membrane filter may be used.

### EXAMPLES

### Example 1

### Effect of high-pressure treatment on dispersibility

After measuring out 0.21 g of mPEG (polyethylene glycol)-PBLA (polyβ(L-aspartic acid)) (polyethylene glycol average molecular weight: 12,000, 50 amino acid residues, PEG-PBLA (12-50)) (Lot M2N531, product of NOF Corp.) in a 100 ml vial, it was dissolved in 100 ml of ultrapure water. The solution was poured into the cell of a microfluidizer (Microfluidics^{™} M-110-EH), the vial was rinsed with 100 ml of ultrapure water, and the rinse solution was also added to the cell of microfluidizer. The conditions for the ultrahigh-pressure dispersion/emulsification treatment with the microfluidizer where changed as shown in Table 1, the particle sizes of the micelles were measured with a NICOMP 380 ZLS, and the chi-square value was calculated as an index of the dispersibility. The results are summarized in Table 1.

The results in Table 1 show that pressurizing treatment of the block copolymer produced a smaller chi-square distribution, especially with an increasing number of treatment times, and resulted in satisfactory block copolymer dispersibility. It was found that a higher pressure in the high-pressure treatment resulted in more satisfactory dispersibility, while a treatment temperature closer to room temperature instead of low temperature resulted in more satisfactory dispersibility.

**Table 1**

| Exp. No. | Cell temperature (°C) | Microfluidizer conditions | | Mean particle size (nm) | S.D. (nm) | Chi-square distribution |
|---|---|---|---|---|---|---|
| | | Pressure (psi) | Treatment times | | | |
| 1 | 23-29 | 20,000 | 10 | 106.5 | 29.8 | 1.180 |
| 2 | 30-31 | 20,000 | 0 | 359.0 | 270.0 | 78.852 |
| | 30-31 | 20,000 | 2 | 120.0 | 28.0 | 1.990 |
| | 30-31 | 20,000 | 4 | 110.6 | 31.2 | 3.894 |
| | 30-31 | 20,000 | 6 | 103.9 | 23.6 | 0.599 |
| | 30-31 | 20,000 | 8 | 103.1 | 24.4 | 1.540 |
| | 30-31 | 20,000 | 10 | 102.1 | 31.4 | 1.818 |
| 3 | 3-5 | 20,000 | 0 | 275.5 | 162.5 | 61.757 |
| | 3-5 | 20,000 | 2 | 124.2 | 31.6 | 1.810 |
| | 3-5 | 20,000 | 4 | 118.6 | 27.5 | 0.612 |
| | 3-5 | 20,000 | 6 | 114.9 | 31.0 | 0.837 |
| | 3-5 | 20,000 | 8 | 114.5 | 31.8 | 0.581 |
| | 3-10 | 20,000 | 10 | 113.8 | 31.5 | 0.444 |
| 4 | 3-5 | 5,000 | 10 | 101.7 | 28.0 | 6.288 |
| | 3-5 | 10,000 | 10 | 92.8 | 31.6 | 4.129 |
| | 3-5 | 20,000 | 10 | 100.3 | 42.4 | 2.137 |

### Example 2

### 1) Preparation of drug (roxithromycin)-encapsulated untreated micelles

After measuring out 49.9 mg of mPEG-PBLA (PEG-PBLA (12-50)) (Lot. M2N531, product of NOF Corp.), it was dissolved in 5 ml of water. Also, 500 µl of previously prepared roxithromycin (Sigma)/dichloromethane (Wako Pure Chemical Industries, Ltd.) (20 mg/ml) was gradually added and the mixture was stirred overnight at 4°C. Upon completion of stirring, the sample was divided into three aliquots as follows: the first was untreated, the second was subjected to sonication (130 W) for 10 minutes, and the third was subjected to sonication (130 W) for 10 minutes followed by filtration with a 0.22 µm membrane filter. Then the particle size of each was measured with a NICOMP 380 ZLS. The sample portion filtered with the 0.22 µm membrane filter was provided for Z-potential measurement, and the amount of drug encapsulation in the micelles with respect to the amount of drug used (10 mg) (encapsulation rate) was also measured. For measurement of the encapsulation rate, the filtered micelles were disrupted with acetonitrile:methanol:0.2 M phosphate buffer (pH 6.1) = 35:35:30, and HPLC was performed using 210 nm absorption as the index.

### 2) Preparation of drug (roxithromycin)-encapsulated pressure-treated micelles

Microfluidizer treatment (20,000 psi, 30°C, 10 times) was carried out in the same manner as Example 1, and 50.4 mg of lyophilized PEG-PBLA (PEG-PBLA (12-50)) was measured out and dissolved in 5 ml of water. Also, 500 µl of previously prepared roxithromycin (Sigma)/dichloromethane (Wako Pure Chemical Industries, Ltd.) (20 mg/ml) was gradually added and the mixture was stirred overnight at 4°C. Upon completion of stirring, the sample was divided into three aliquots as follows: the first was untreated, the second was subjected to sonication (130 W) for 10 minutes, and the third was subjected to sonication (130 W) for 10 minutes followed by filtration with a 0.22 µm membrane filter. Then the particle size of each was measured with a NICOMP 380 ZLS. The sample portion filtered with the 0.22 µm membrane filter was provided for Z-potential measurement, and the encapsulation rate was also measured in the same manner as described above.

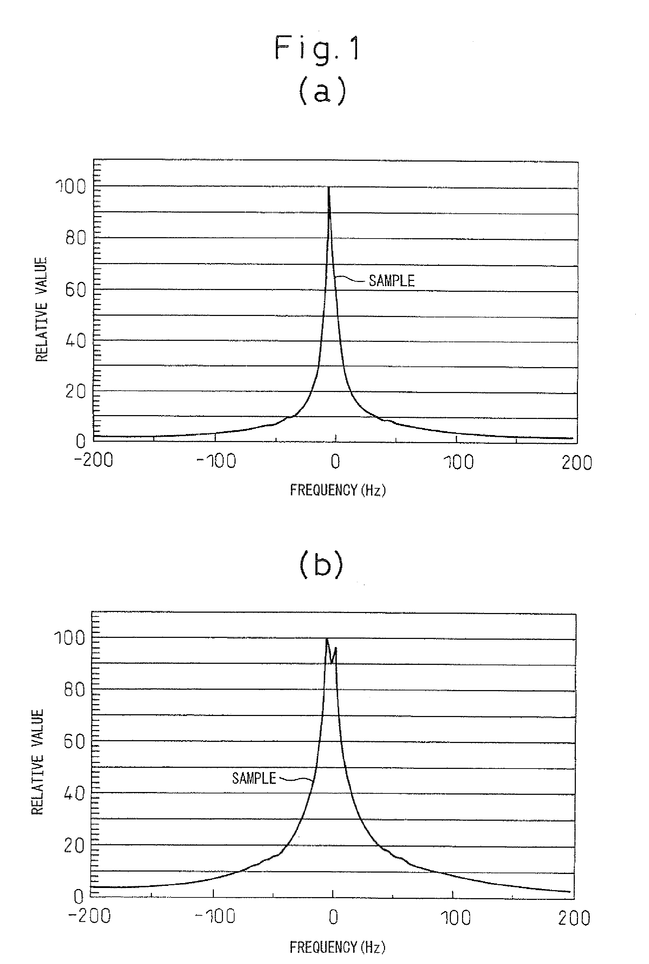

The results of measuring the drug-encapsulated micelle particle sizes are shown in Table 2, and the results of Z-potential measurement are shown in Fig. 1. The drug encapsulation rates are shown in Table 3. The results shown in Table 2 demonstrate that the pressure-treated micelles had more satisfactory dispersibility than the untreated micelles even with actual encapsulation of a drug agent. The Z-potential measurement results in Fig. 1 indicate that the drug-encapsulating and treated micelles showed only one peak (a) while the drug-encapsulated untreated micelles showed two peaks (b). This suggests that using an untreated block copolymer for encapsulation of a drug largely results in formation of micelles with two different drug concentrations. In contrast, using a pressurizing treated block copolymer for encapsulation of a drug gives a single peak, thus suggesting that micelles with only a single drug concentration are formed. The filterability of the drug-encapsulated pressure-treated micelles was highly satisfactory compared to the drug-encapsulated untreated micelles. Also, the drug encapsulation rate of the drug-encapsulated pressure-treated polymer micelles was superior to that of the untreated micelles.

**Table 2**

| Condition | Gaussian distribution | | | | NICOMP distribution | | |
|---|---|---|---|---|---|---|---|
| | Strength | | Volume | Number | Volume peak position (nm) | | |
| | Mean (S.D.) | Chi-square distribution | Mean (S.D.) | Mean (S.D.) | Peak 1 (%) | Peak 2 (%) | Peak 3 (%) |
| Untreated/without drug | 359.0 [270.0] | 78.85 | 388.9 [292.5] | 49.3 | 19.6 [54.3] | 170.8 [7.9] | 1557.6 [37.8] |
| Treated/with drug | 103.1 [24.4] | 1.54 | 89.4 [21.2] | 74.8 [17.7] | 22.3 [46.5] | 99.3 [53.5] | |
| Untreated/with drug | 167.6 [85.2] | 12.22 | 104.2 [52.9] | 38.9 [19.7] | 37.6 [63.1] | 152.0 [28.3] | 1625.6 [8.6] |
| Untreated/with drug Sonication | 96.1 [19.3] | 1.31 | 86.5 [17.4] | 76.3 [15.3] | 25.1 [24.9] | 90.1 [75.1] | |
| Untreated/with drug Sonication/filtration treatment | 94.8 [16.1] | 1.53 | 88.0 [15.0] | 80.5 [13.7] | 87.1 [100.0] | - | - |
| Treated/with drug | 130.6 [50.4] | 1.34 | 84.0 [36.3] | 43.8 [18.9] | 11.3 [82.9] | 105.3 [15.3] | 293.7 [1.1] |
| Treated/with drug Sonication | 95.8 [27-1] | 0.46 | 77.5 [21.9] | 60.1 [17.0] | 79.3 [100.0] | - | - |
| Treated/with drug Sonication + filtration treatment | 93.9 [20.7] | 0.68 | 82.7 [18.2] | 71.1 [15.6] | 87.5 [100.0] | - | - |

**Table 3**

| Polymer | Encapsulation rate |
|---|---|
| Non-pressure-treated polymer | 53% |
| Pressure-treated polymer | 72% |

### Example 3

### State of pressure-treated block copolymer solution

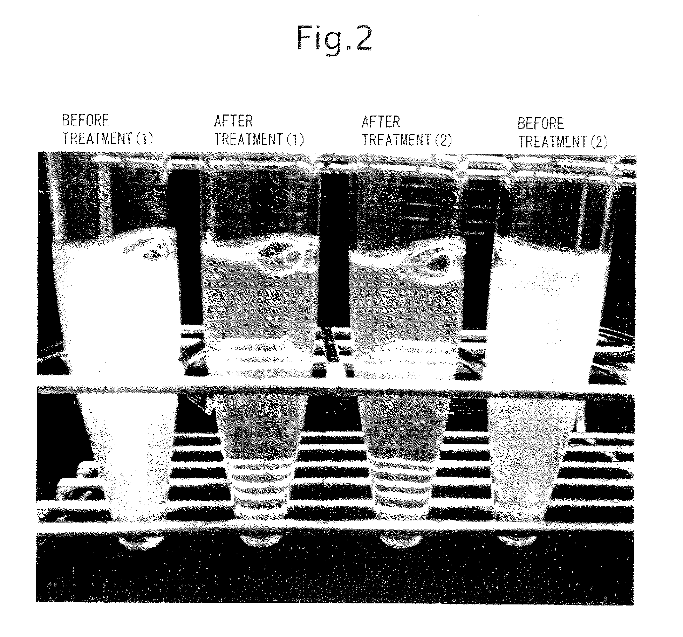

After measuring out 30 mg of PEG-PBLA (PEG-PBLA (12, 50)) subjected to microfluidizer treatment (137.8 MPa (20,000 psi), 30°C, 17 times) in the manner described in Example 1, or of untreated PEG-PBLA, it was dissolved in 3 ml of water and stirred at room temperature for 1 hour, after which the state of the solution was visually examined. Fig. 2 is a photograph showing the solutions before and after pressure treatment. The labels (1) and (2) in the photograph indicate different lots. This photograph shows that subjecting the block copolymer to pressure treatment can significantly reduce solution turbidity.

## Claims

1. A pharmaceutical formulation comprising drug-encapsulated polymer micelles formed from a block copolymer having a hydrophilic segment and hydrophobic segment, and has been subjected to high-pressure treatment.

2. A pharmaceutical formulation according to claim 1, wherein the high-pressure treatment is carried out by a process including a step in which a solution containing the block copolymer is subjected to ultrahigh-pressure dispersion/emulsification.

3. A pharmaceutical formulation according to claim 2, wherein the ultrahigh-pressure dispersion/emulsification is carried out with an ultrahigh-pressure homogenizer.

4. A pharmaceutical formulation according to any one of claims 1 to 3, wherein the pressure for the high-pressure treatment is 3,000 MPa or greater.

5. A pharmaceutical formulation according to claim 4, wherein the pressure for the high-pressure treatment is 20,000 MPa or greater.

6. A pharmaceutical formulation according to any one of claims 1 to 5, wherein the block copolymer which has a higher-order structure is subjected to high-pressure treatment to yield a polymer with the higher-order structure of the polymer destroyed.

7. A pharmaceutical formulation according to any one of claims 1 to 5, wherein the block copolymer which has a higher-order structure and is biocompatible is subjected to high-pressure treatment to yield a polymer with the higher-order structure of the polymer destroyed.

8. A pharmaceutical formulation according to any one of claims 1 to 7, wherein the hydrophobic segment is a polyamino acid derivative.

9. A pharmaceutical formulation according to claim 8, wherein the polyamino acid derivative is a poly(aspartic acid) derivative.

10. A pharmaceutical formulation according to claim 9, wherein the polyamino acid derivative is a poly(glutamic acid) derivative.

11. A pharmaceutical formulation according to any one of claims 1 to 7, wherein the hydrophobic segment is a biocompatible polymer or biodegradable polymer.

12. A pharmaceutical formulation according to any one of claims 1 to 11, wherein the hydrophilic segment is polyethylene glycol or a derivative thereof.

13. A pharmaceutical formulation according to any one of claims 1 to 12, wherein the hydrophilic segment has 30-1000 repeating units and the hydrophobic segment has 10-100 repeating units.

14. A pharmaceutical formulation according to any one of claims 1 to 13, which contains a drug selected from the group of drugs consisting of anticancer agents, immunosuppressive agents, antibiotics, antirheumatic drugs, antimicrobial agents, antihypertensive drugs, central nervous system acting drugs, hormone agents, diabetes drugs, anti-inflammatory drugs, analgesics, antiviral agents, antimalarial drugs, biopreparations, gene therapy agents such as DNA or RNA, antibody agents, proteins and peptides.

15. A pharmaceutical formulation according to any one of claims 1 to 13, which comprises a drug selected from the group consisting of roxithromycin, paclitaxel, topotecan, camptothecin, cisplatin, daunorubicin hydrochloride, methotrexate, mitomycin C, docetaxel, vincristine sulfate, polyene antibiotics, amphotericin B, nystatin and prostaglandins.

16. A treatment method for a block copolymer having a hydrophilic segment and a hydrophobic segment for formation of drug-encapsulated polymer micelles, the treatment method comprises a step of subjecting the block copolymer to high-pressure treatment.

17. A method according to claim 16, wherein the high-pressure treatment is carried out by a process including a step in which a solution containing the block copolymer is subjected to ultrahigh-pressure dispersion/emulsification.

18. A method according to claim 17, wherein the ultrahigh-pressure dispersion/emulsification is carried out with an ultrahigh-pressure homogenizer.

19. A method according to any one of claims 16 to 18, wherein the pressure for the high-pressure treatment is 3,000 MPa or higher.

20. A method according to claim 19, wherein the pressure for the high-pressure treatment is 20,000 MPa or higher.

21. A method according to any one of claims 16 to 20, wherein the block copolymer which has a higher-order structure is subjected to high-pressure treatment to yield a polymer with the higher-order structure of the polymer destroyed.

22. A method according to any one of claims 16 to 20, wherein the block copolymer which has a higher-order structure and is biocompatible is subjected to high-pressure treatment to yield a polymer with the higher-order structure of the polymer destroyed.

23. A method according to any one of claims 16 to 22, wherein the hydrophobic segment is a polyamino acid derivative.

24. A method according to claim 23, wherein the polyamino acid derivative is a poly(aspartic acid) derivative.

25. A method according to claim 24, wherein the polyamino acid derivative is a poly(glutamic acid) derivative.

26. A method according to any one of claims 16 to 22, wherein the hydrophobic segment is a biocompatible polymer or a biodegradable polymer.

27. A method according to any one of claims 16 to 26, wherein the hydrophilic segment is polyethylene glycol or a derivative thereof.

28. A method according to any one of claims 16 to 27, wherein the hydrophilic segment has 30-1000 repeating units and the hydrophobic segment has 10-100 repeating units.

29. A method according to any one of claims 16 to 28, which includes the use of a drug selected from the group of drugs consisting of anticancer agents, immunosuppressive agents, antibiotics, antirheumatic drugs, antimicrobial agents, antihypertensive drugs, central nervous system acting drugs, hormone agents, diabetes drugs, anti-inflammatory drugs, analgesics, antiviral agents, antimalarial drugs, biopreparations, gene therapy agents such as DNA or RNA, antibody agents, proteins and peptides.

30. A method according to any one of claims 16 to 28, which includes the use of a drug selected from the group consisting of roxithromycin, paclitaxel, topotecan, camptothecin, cisplatin, daunorubicin hydrochloride, methotrexate, mitomycin C, docetaxel, vincristine sulfate, polyene antibiotics, amphotericin B, nystatin and prostaglandins.
